# EUROPEAN PATENT APPLICATION

(11) **EP 1 745 735 A1**
(43) Date of publication of application: **24.01.2007**
(21) Application number: 05703836.6
(22) Date of filing: 19.01.2005
(51) Int. Cl.: A61B 1/00

(54) **ELECTRONIC ENDOSCOPE AND ENDOSCOPE SYSTEM USING THE SAME**

(30) Priority: 20.01.2004 JP 2004012075
(71) Applicant: TEXAS INSTRUMENTS JAPAN LTD., Shinjuki-ku, Tokyo 160-8366 (JP)
(72) Inventor: KASHIMA, Shunji c/o Texas Instruments Japan Ltd., Ibaraki 300-0496 (JP)
(74) Representative: Holt, Michael
(86) International application number: PCT/JP2005/000599
(87) International publication number: WO 2005/067784

(57) **Abstract**

The objective of the invention is to provide a type of electronic endoscope that can reduce the noise in the image pickup signal sent from the electronic endoscope, and a type of endoscope system using said electronic endoscope. Electronic endoscope 1 has solid-state image pickup element 12, A/D converter 30 that converts the analog image pickup signal from solid-state image pickup element 12 to a digital signal, and parallel/series converter 32 that converts the parallel input digital signal from A/D converter 30 to a series signal. The digitally converted image pickup signal is sent from electronic endoscope 1, so that even when cable 58 becomes longer, the noise resistance can still be improved by comparison to the analog signal in the prior art.

## Description

### FIELD OF THE INVENTION

The present invention pertains to a type of electronic endoscope and an endoscope system using said electronic endoscope. In particular, the present invention pertains to a type of circuit constitution contained in the electronic endoscope.

### BACKGROUND OF THE INVENTION

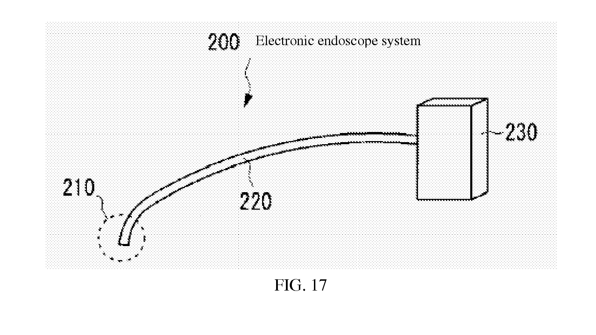

Ultra-small CCD and CMOS sensors have been developed as solid-state image pickup elements that have been used in electronic endoscopes in the medical field. The electronic endoscope is inserted into the human body to take pictures of diseased areas, etc. Consequently, there is a demand for miniaturization. For example, as shown in FIG. 16, image pickup device 100 disclosed in Patent Reference 1 has optical glass 110, CCD chip 120 and laminated circuit board 130. CCD 120 is connected to the wiring of laminated circuit board 130 by means of tab terminals 122. Cavity 132 is formed in laminated circuit board 130. Small chip component 140 is assembled in this cavity 132. Large chip component 150 is assembled above cavity 132. An ultra-small image pickup device 100 is realized with this constitution. Also, chip components 140, 150 are a transistor and capacitor that process the image pickup signal from the CCD.

Also, in the scheme described in Patent Reference 2, the electronic component assembled on the circuit board of the image pickup device is protected from environmental influences, such as mechanical and electric shocks, air, thermal shocks, etc., by improving the viscosity of the sealant of the electronic component assembled on the circuit board.

FIG. 17 is a diagram illustrating the schematic constitution of the electronic endoscope system. Electronic endoscope system 200 has electronic endoscope 210 and external controller 230 that is connected to electronic endoscope 210 via cable 220. Electronic endoscope 210 forms the so-called scope tip portion. This portion is inserted into the human body to take pictures of the target diseased area. External controller 230 forms the so-called scope terminal portion, and it receives the signal picked up by electronic endoscope 210 via cable 220, this signal being displayed on a display unit or the like. Also, the control signals for controlling the operation of electronic endoscope 210 are sent via cable 220. At the scope tip, in addition to the electronic endoscope, there is an operating part for cutting and harvesting cells, polyps, or the like from the diseased area, and the operating part is subjected to remote control by external controller 230.

Patent Reference 1: Japanese Kokai Patent Application No. 2002-76314

Patent Reference 2: Japanese Kokai Patent Application No. Hei 8[1996]-146308

However, said electronic endoscope and electronic endoscope system of the prior art have the following problem. As shown in FIG. 17, electronic endoscope 200 has a constitution in which the analog signal picked up electronic endoscope 210 is sent through cable 220 to external controller 230. Consequently, as cable 220 becomes longer, the noise level in the analog signal becomes higher. As a result, the image of the diseased area displayed on the display unit by external controller 130 degrades, and this is undesirable.

The purpose of the present invention is to solve the aforementioned problems of the prior art by providing a type of electronic endoscope, and an endoscope system using the electronic endoscope, that can reduce the noise in the pickup signal sent from the electronic endoscope.

### SUMMARY OF THE INVENTION

The present invention provides a type of electronic endoscope characterized by the following facts: the electronic endoscope has a solid-state image pickup element, a circuit board having at least one semiconductor device assembled on it for processing the signal from the solid-state image pickup element, and a connecting terminal for electrical connection of the semiconductor device assembled on the circuit board to an external controller; said at least one semiconductor device has a digital converter that converts the analog image pickup signal from the solid-state image pickup element to a digital image pickup signal, and a circuit that sends the digitally converted image pickup signal to the external controller via said connecting terminal.

It is preferred that said circuit contain a parallel/series converter that converts the parallel input digital signal to a serial signal. Also, said parallel/series converter converts the digital signal input in series form from the external controller to a parallel signal.

It is preferred that said at least one semiconductor device contain an amplifier that amplifies the analog image pickup signal from the solid-state image pickup element, and the analog image pickup signal amplified by said amplifier is sent to said digital converter.

It is preferred that said at least one semiconductor device contain a controller electrically connected to said parallel/series converter. Said controller may control the solid-state image pickup element, and/or the amplifier and/or the digital converter on the basis of the control signals output from said parallel/series converter.

It is only necessary that said at least one semiconductor circuit contain a driver circuit for driving the solid-state image pickup element, and the driver circuit can operate according to the control signals from said controller.

It is preferred that said at least one semiconductor device contain an oscillator that generates a clock signal. Said controller may output the control signals in response to the clock signal from the oscillator.

The electronic endoscope system of the present invention contains said electronic endoscope and an external controller that is electrically connected via a cable to the connecting terminal of the electronic endoscope. Said external controller has a series/parallel converter that converts series input digital signals to a parallel output. The series/parallel converter sends series converted digital signals via said cable to the parallel/series converter of the electronic endoscope, and it receives the series converted digital signals from the parallel/series converter of the electronic endoscope via said cable.

### BRIEF DESCRIPTION OF THE DRAWINGS

FIG. 1 is a diagram illustrating the constitution of the electronic endoscope in an embodiment of the present invention.
FIG. 2 is a block diagram illustrating the electrical constitution of the electronic endoscope in Embodiment 1 of the present invention.
FIG. 3 is a diagram illustrating another constitution of the electronic endoscope.
FIG. 4 is a block diagram illustrating the electrical constitution of the electronic endoscope in Embodiment 2 of the present invention.
FIG. 5 is a block diagram illustrating the electrical constitution of the electronic endoscope in Embodiment 3 of the present invention.
FIG. 6 is a block diagram illustrating the electrical constitution of the electronic endoscope in Embodiment 4 of the present invention.
FIG. 7 is a block diagram illustrating the electrical constitution of the electronic endoscope in Embodiment 5 of the present invention.
FIG. 8 is a block diagram illustrating the electrical constitution of the electronic endoscope in Embodiment 6 of the present invention.
FIG. 9 is a block diagram illustrating the electrical constitution of the electronic endoscope in Embodiment 7 of the present invention.
FIG. 10 is a block diagram illustrating the electrical constitution of the electronic endoscope in Embodiment 8 of the present invention.
FIG. 11 is a block diagram illustrating the electrical constitution of the electronic endoscope in Embodiment 9 of the present invention.
FIG. 12 is a block diagram illustrating the electrical constitution of the electronic endoscope in Embodiment 10 of the present invention.
FIG. 13 is a block diagram illustrating the electrical constitution of the electronic endoscope in Embodiment 11 of the present invention.
FIG. 14 is a block diagram illustrating the electrical constitution of the electronic endoscope in Embodiment 12 of the present invention.
FIG. 15 is a block diagram illustrating another example of the constitution of the electronic endoscope of the present invention.
FIG. 16 is a diagram illustrating the constitution of the electronic endoscope in the prior art.
FIG. 17 is a diagram illustrating the constitution of the electronic endoscope system.

### REFERENCE NUMERALS A AND SYMBOLS S SHOWN IN THE DRAWINGS

1 is an electronic endoscope, 12 a solid-state image pickup element, 30 an A/D converter, 32 a parallel/series converter, 34 a control unit, 50 an external controller, 52 a power supply, 54 a series/parallel converter, 56 a DSP, 58 a cable, 60 a power line, 62 a signal line, and 64 a signal line.

### DESCRIPTION OF THE EMBODIMENTS

According to the present invention, the electronic endoscope converts the analog image pickup signal to a digital signal for transmission. Consequently, it is possible to improve the noise resistance of the image pickup signal sent from the electronic endoscope. As a result, even when the wiring of the cable that connects the electronic endoscope to the external controller is long, it is still possible to display the picked-up image with high image quality on the display unit.

In the following, an explanation will be given regarding a preferred embodiment of the present invention.

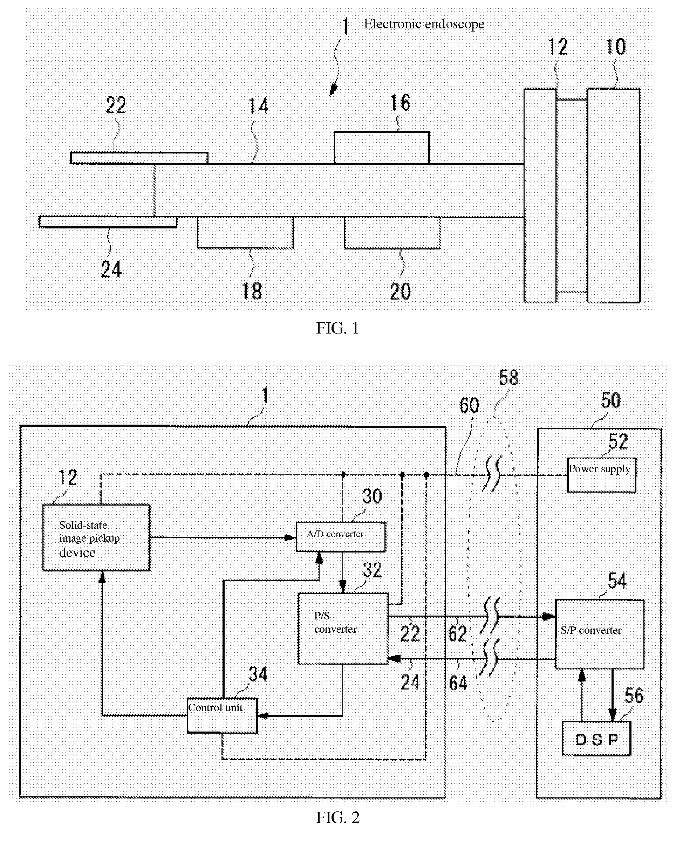

FIG. 1 is a diagram illustrating schematically the constitution of the electronic endoscope in an embodiment of the present invention. As shown in the figure, electronic endoscope 1 is composed of protective glass 10, a CCD or CMOS sensor or other solid-state image pickup element 12, and circuit board 14. Solid-state image pickup element 12 is electrically connected to the prescribed circuit wiring of the circuit board using the same tab terminals or the like as in the prior art. Plural semiconductor devices 16, 18, 20 are assembled on opposite surfaces of circuit board 14. It is preferred that semiconductor devices 16, 18, 20 each be composed of wafer level CSPs (chip size packages). They are electrically connected to the prescribed circuit wiring of circuit board 14. As will be explained later, these semiconductor devices contain circuits for processing the picked-up signals from solid-state image pickup element 12, and for processing the signals sent from the external controller.

A pair of connecting terminals 22, 24 are connected to the terminal portions of circuit board 14. One end of said pair of connecting terminals 22, 24 is electrically connected to the prescribed circuit wiring on the circuit board, and the other end portion is electrically connected to the cable connected to the external controller (see FIG. 2). Said protective glass 10, solid-state image pickup element 12, circuit board 14, semiconductor devices 16, 18, 20, and connecting terminals 22, 24 are accommodated together with optical lenses in a cylindrical casing not shown in the figure, and the interior of the casing is filled with a sealant.

FIG. 2 is a block diagram illustrating the electrical constitution of electronic endoscope 1 in Embodiment 1. As shown in this figure, electronic endoscope 1 is composed of solid-state image pickup element 12, A/D converter 30 that receives the analog image pickup signal from solid-state image pickup element 12 and converts it to a digital image pickup signal, parallel/series converter 32, and control part 34. It is preferred that said A/D converter 30 comprise the circuit of semiconductor device 16, and parallel/series converter 32 comprise the circuit of semiconductor device 18. Control part 34 comprises the circuit of semiconductor device 20. For example, it may consist of an FPGA, etc.

Parallel/series converter 32 converts the digital signal parallel input from A/D converter 30 to a series output, and it sends the obtained signal to connecting terminal 22. Also, it converts the digital signal input in series from connecting terminal 24 to a parallel output, and outputs it to control part 34. Control part 34 receives the digital signal from parallel/series converter 32, and it controls the operation of A/D converter 30 and solid-state image pickup element 12 in response to this signal.

On the other hand, external controller 50 in the scope terminal portion is electrically connected via cable 58 to electronic endoscope 1. External controller 50 is composed of power supply 52, parallel/series converter 54, DSP 56, etc. Electronic endoscope 1 and external controller 50 are electrically connected to each other via cable 58. Cable 58 is connected to power supply 52, and it contains power line 60 for supplying electrical power, and signal lines 62, 64 connected to connecting terminals 22, 24. In the figure, power line 60 is represented by a broken line to distinguish it from the signal lines.

Series/parallel converter 54 converts the digital signal parallel input from DSP 56 to a series output, which is sent via cable 58 to connecting terminal 24. It also receives the series converted digital signal from parallel/series converter 32 via the cable, converts it to a parallel output, and sends it to DSP 56.

DSP 56 performs image processing of the digital image pickup signal fed from electronic endoscope 1, and it generates the digital control signals for controlling the operation of electronic endoscope 1. Also, external controller 50 is connected to another external controller, and it outputs the digital image pickup signal to said other external controller and controls electronic endoscope 1 in response to the digital control signals from said other external controller.

For example, DSP 56 is used to generate the digital control signals needed to control the exposure and driving timing, etc., of solid-state image pickup element 12, and these signals are parallel input to series/parallel converter 54. Series/parallel converter 54 converts the parallel input digital control signal to a series output. The converted digital signal is sent via signal line 64 of cable 58 and connecting terminal 24 to parallel/series converter 32. Parallel/series converter 32 converts the series input digital control signal to a parallel output that is sent to control part 34. Control unit 34 controls solid-state image pickup element 12 and A/D converter 30 on the basis of the input digital control signal, and it adjusts the exposure and driving timing.

On the other hand, the analog image pickup signal from solid-state image pickup element 12 is input to A/D converter 30 and is converted by it to a digital image pickup signal. The digitally converted image pickup signal is parallel input to parallel/series converter 34, and is converted by it to a series output. The converted digital signal is sent through signal line 62 of cable 58 and is series input to series/parallel converter 54, which converts it to a parallel output and sends it to DSP 56. DSP 56 performs the necessary signal processing based on the received digital image pickup signal.

In this way, according to the present embodiment, electronic endoscope 1 converts the analog image pickup signal to a digital signal and sends out the digital signal. Consequently, compared with the prior art in which an analog image pickup signal is sent out, it is possible to have high noise resistance even when cable 58 becomes longer.

Also, solid-state image pickup element 12 may contain a circuit that performs CDS correlated double sampling to remove noise from the image pickup signal. In addition, one may also adopt a scheme in which external controller 50 and electronic endoscope 1 have a master/slave relationship, and the digital signal from series/parallel converter 54 with a clock signal superimposed on it is output to parallel/series converter 32, and control unit 34 performs control of the various parts by means of timing in response to the clock signal. Said parallel/series converter 32 and series/parallel converter 54 may comprise a serializer/deserializer.

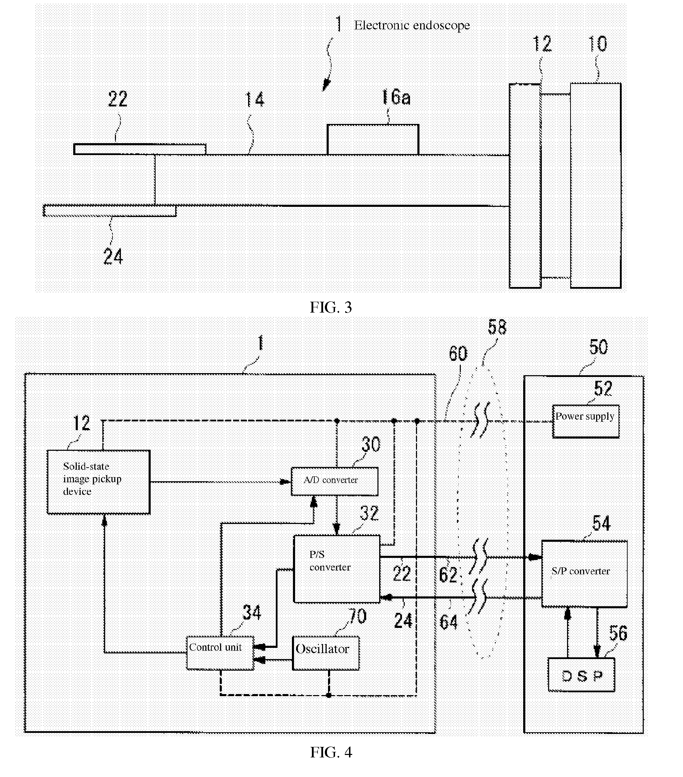

FIG. 3 is a diagram illustrating another example of the constitution of the electronic endoscope. In the example shown in FIG. 1, three semiconductor devices are assembled on the circuit board, and they form said A/D converter 30, parallel/series converter 32, and control unit 34. However, there is no special limitation on the form. As shown in FIG. 3, one may also adopt a scheme in which said plural circuits are contained in single semiconductor device 16a assembled on circuit board 14. Also, single semiconductor device 16a may consist of a single semiconductor chip or a multi-chip module consisting of plural semiconductor chips.

FIG. 4 is a block diagram that illustrates the electrical constitution of the electronic endoscope pertaining to Embodiment 2 of the present invention. In addition to the constitution of Embodiment 1, the electronic endoscope in Embodiment 2 also has oscillator 70. In Embodiment 2, control part 34 controls the operation of A/D converter 30 and solid-state image pickup element 12 on the basis of the clock signal generated by oscillator 70. As a result, there is no need to feed a clock signal from external controller 50 to electronic endoscope 1.

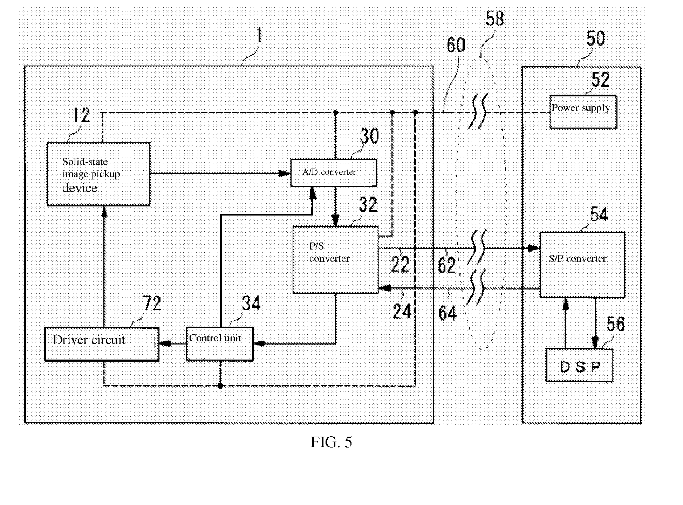

FIG. 5 is a block diagram illustrating the electrical constitution of the electronic endoscope in Embodiment 3 of the present invention. In addition to the constitution of said Embodiment 1, the electronic endoscope in Embodiment 3 also has driver circuit 72. Driver circuit 72 responds to the control signals from control part 34 and drives solid-state image pickup element 12 with a prescribed driving voltage. Also, when solid-state image pickup element 12 consists of a CMOS sensor, it is not necessary to drive by means of driver circuit 72 since the swing width of the driving voltage is small.

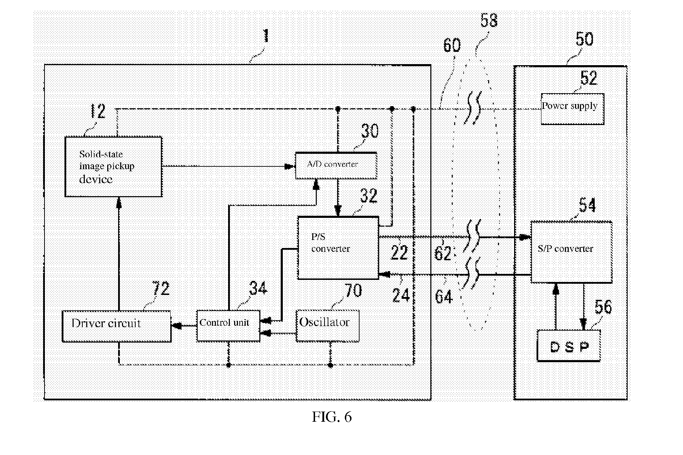

FIG. 6 is a block diagram illustrating the electrical constitution of the electronic endoscope in Embodiment 4 of the present invention. The constitution of Embodiment 4 includes both Embodiments 2 and 3, that is, it has oscillator 70 and driver circuit 72.

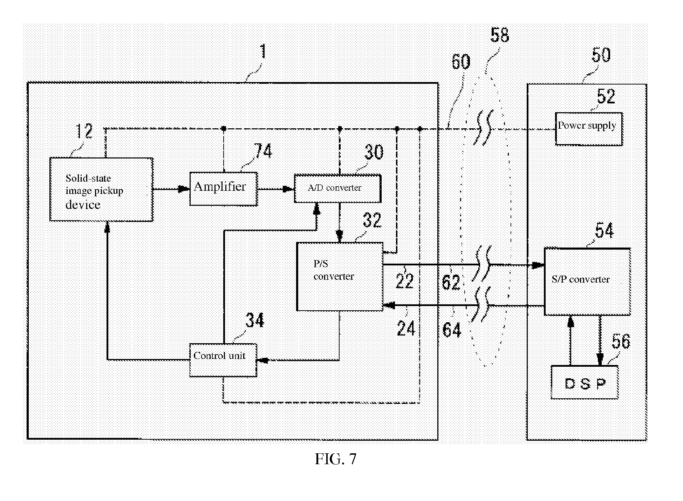

FIG. 7 is a block diagram illustrating the electrical constitution of the electronic endoscope in Embodiment 5 of the present invention. In addition to the constitution of Embodiment 1, Embodiment 5 also has amplifier 74. Amplifier 74 amplifies the analog image pickup signal from solid-state image pickup 12, and it is output to A/D converter 30. As explained above, when solid-state image pickup element 12 performs correlated double sampling, amplifier 74 may have plural sections of source follower type amplifiers connected to each other, and it may contain a differential type amplifier.

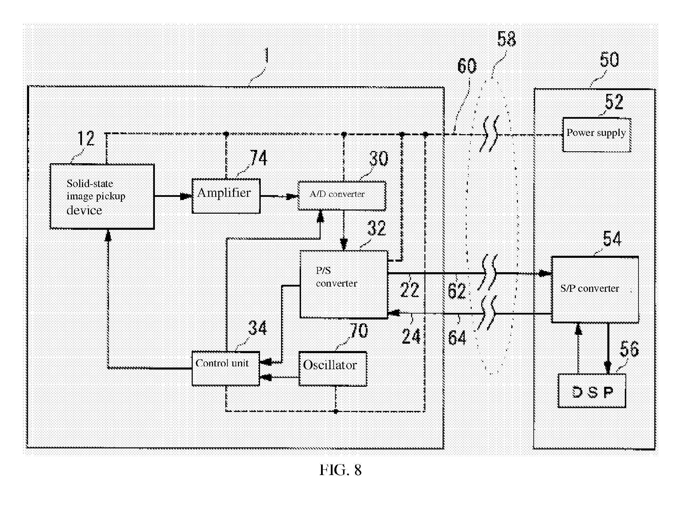

FIG. 8 is a block diagram illustrating the electrical constitution of the electronic endoscope in Embodiment 6 of the present invention. In addition to the constitution of Embodiment 1, Embodiment 6 also has amplifier 70 and amplifier 74.

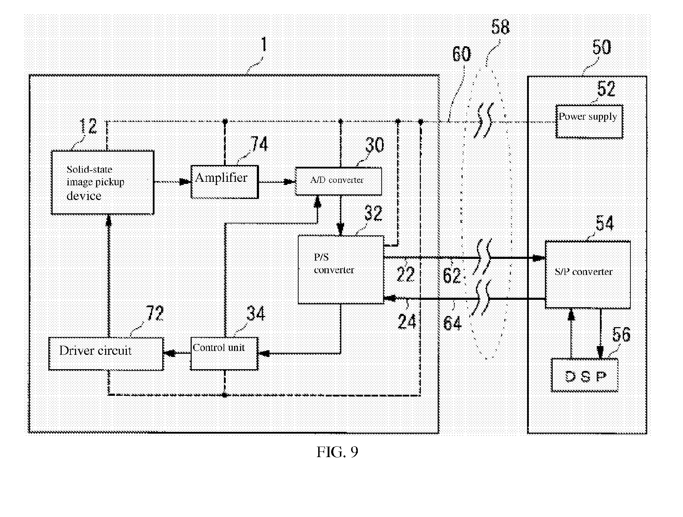

FIG. 9 is a block diagram illustrating the electrical constitution of the electronic endoscope in Embodiment 7 of the present invention. In addition to the constitution of Embodiment 1, Embodiment 7 also has driver circuit 72 and amplifier 74.

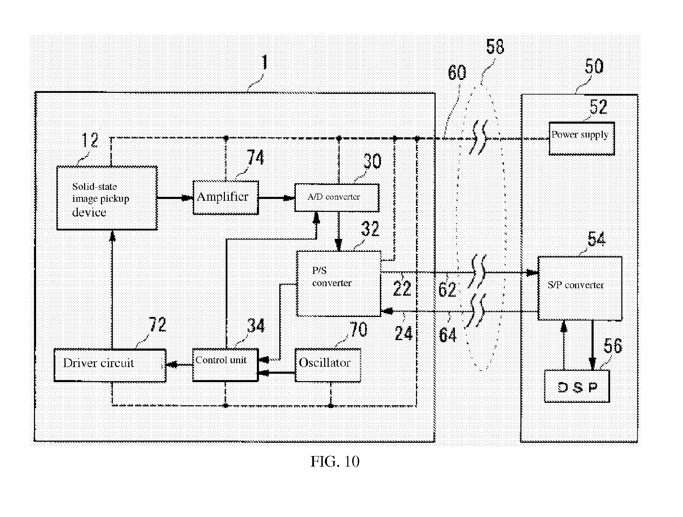

FIG. 10 is a block diagram illustrating the electrical constitution of the electronic endoscope in Embodiment 8 of the present invention. In addition to the constitution of Embodiment 1, Embodiment 8 also has oscillator 70, driver circuit 72 and amplifier 74.

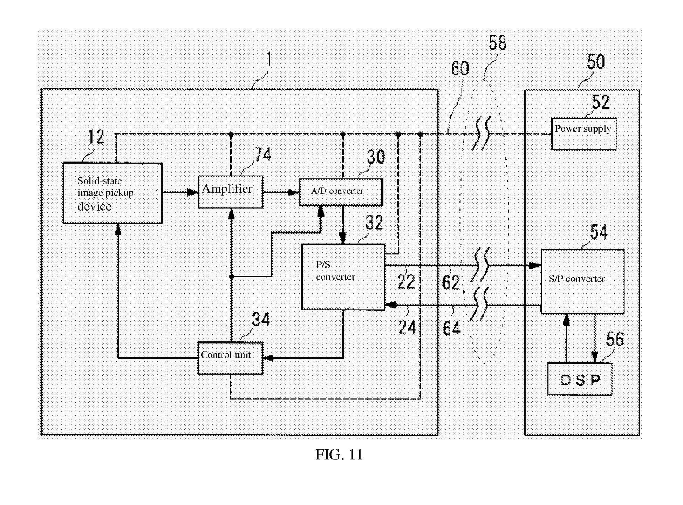

FIG. 11 is a block diagram illustrating the electrical constitution of the electronic endoscope in Embodiment 9 of the present invention. In addition to the constitution of Embodiment 1, Embodiment 9 has amplifier 74, and the operation of amplifier 74 is controlled in response to control signals from control unit 34.

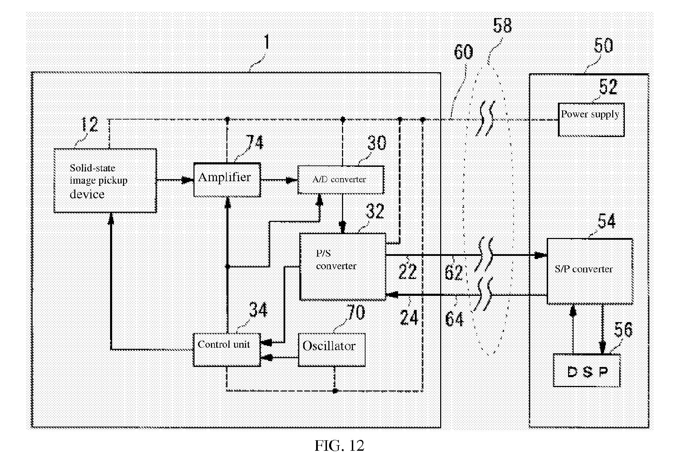

FIG. 12 is a block diagram illustrating the electrical constitution of the electronic endoscope in Embodiment 10 of the present invention. In addition to the constitution of Embodiment 9, Embodiment 10 also has oscillator 70.

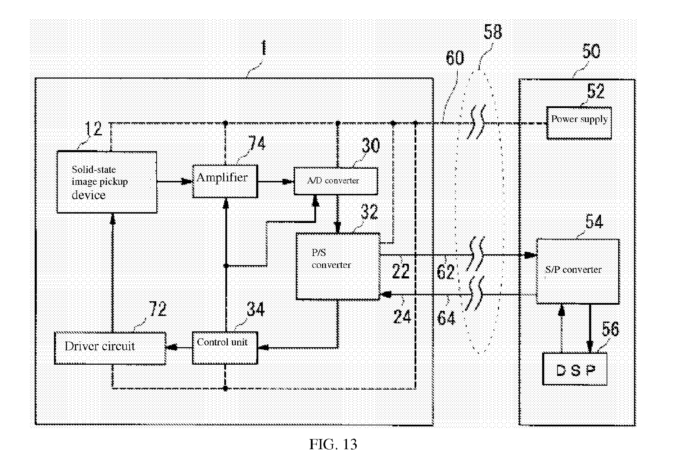

FIG. 13 is a block diagram illustrating the electrical constitution of the electronic endoscope in Embodiment 11 of the present invention. In addition to the constitution of Embodiment 9, Embodiment 11 has driver circuit 72.

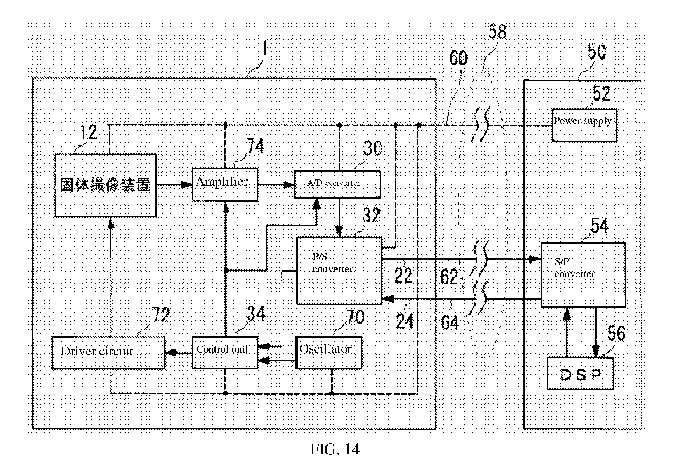

FIG. 14 is a block diagram illustrating the electrical constitution of the electronic endoscope in Embodiment 12 of the present invention. In addition to the constitution of Embodiment 9, Embodiment 12 has oscillator 70 and driver circuit 72.

The circuits of said Embodiments 2-12 each may be formed as a single semiconductor device 16a or may be divided into plural semiconductor devices.

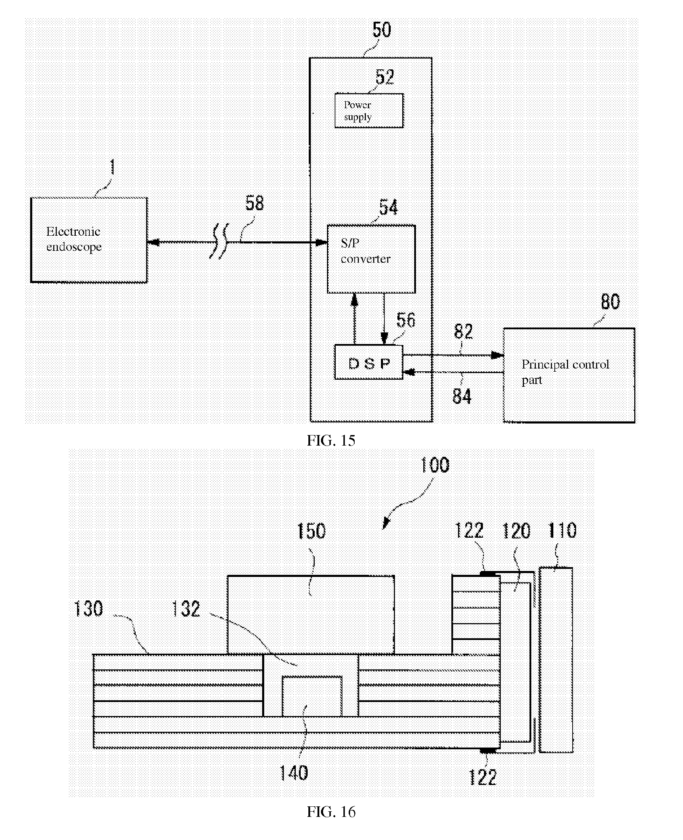

In said Embodiments 1-12, electronic endoscope 1 is formed at the tip portion of the scope, and external controller 50 forms the scope tail portion. However, the electronic endoscope system is not limited to this constitution. For example, as shown in FIG. 15, one may also use another principal controller 80 connected to external controller 50 to control external controller 50 and electronic endoscope 1 with principal controller 80. For example, one may also adopt a scheme in which control signal 82 for external controller 50 for image processing is sent from principal controller 80 to DSP 56, and, on the other hand, signal 84 that has been subjected to image processing is sent from DSP 56 to principal controller 80.

In the above, the preferred embodiments of the present invention have been explained in detail. However, the present invention is not limited to the aforementioned embodiments. Various modifications and changes can be made as long as the gist of the present invention described in the claims is observed.

The electronic endoscope and the electronic endoscope system of the present invention are used in medical applications, as well as in places where direct viewing is difficult, such as in pipelines, in small spaces, or in machine equipment that may be dangerous to the human body.

## Claims

1. A type of electronic endoscope **characterized by** the following facts: the electronic endoscope has a solid-state image pickup element, a circuit board having at least one semiconductor device assembled on it for processing the signal from the solid-state image pickup element, and a connecting terminal for electrical connection of the semiconductor device assembled on the circuit board to an external controller; said at least one semiconductor device has a digital converter that converts the analog image pickup signal from the solid-state image pickup element to a digital image pickup signal, and a circuit that sends the digitally converted image pickup signal to the external controller via said connecting terminal.

2. The electronic endoscope described in Claim 1, **characterized by** the fact that said circuit contains a parallel/series converter that converts the parallel input digital signal to a serial signal.

3. The electronic endoscope described in Claim 2, **characterized by** the fact that said parallel/series converter converts the digital signal input in series form from the external controller to a parallel signal.

4. The electronic endoscope described in Claim 1 or 2, **characterized by** the fact that said at least one semiconductor device contains an amplifier that amplifies the analog image pickup signal from the solid-state image pickup element, and the analog image pickup signal amplified by said amplifier is sent to said digital converter.

5. The electronic endoscope described in any of Claims 1-4, **characterized by** the fact that said at least one semiconductor device contains a controller electrically connected to said parallel/series converter, and said controller controls the solid-state image pickup element, and/or the amplifier, and/or the digital converter on the basis of the control signal outputs from said parallel/series converter.

6. The electronic endoscope described in Claim 5, **characterized by** the fact that said at least one semiconductor circuit contains a driver circuit for driving the solid-state image pickup element, and the driver circuit operates according to the control signals from said controller.

7. The electronic endoscope described in Claim 4, **characterized by** the fact that said at least one semiconductor device contains an oscillator that generates a clock signal, and said controller outputs the control signals in response to the clock signal from the oscillator.

8. The electronic endoscope described in on of Claims 1-7, **characterized by** the fact that said at least one semiconductor device contains plural semiconductor devices assembled on at least one of the opposite surfaces of the circuit board.

9. The electronic endoscope described in one of Claims 1-8, **characterized by** the fact that said solid-state image pickup element contains a CCD or CMOS sensor.

10. A type of electronic endoscope system **characterized by** the following facts:
it contains the electronic endoscope described in one of Claims 1-9,
and an external controller that is electrically connected via a cable to the connecting terminal of the electronic endoscope;
said external controller has a series/parallel converter that converts the series input digital signal to a serial output or the series input digital signal to a parallel output; the second series/parallel converter sends the series converted digital signal to the parallel/series converter of the electronic endoscope via said cable, and it receives the series converted digital signal from the parallel/series converter of the electronic endoscope via said cable.

11. The electronic endoscope system described in Claim 10, **characterized by** the fact that said electronic endoscope forms a scope tip portion, and said external controller forms a scope tail portion.
